# EUROPÄISCHE PATENTANMELDUNG

(11) **EP 2 383 245 A2**
(43) Veröffentlichungstag der Anmeldung: **02.11.2011**
(21) Anmeldenummer: 11162982.0
(22) Anmeldetag: 19.04.2011
(51) Int. Cl.: C07B 45/04, C07C 315/02, C07C 317/14, C07C 317/22

(54) **Verfahren zur kontinuierlichen Oxidation von Thioethern**

(30) Priorität: 20.04.2010 DE 102010015529
(71) Anmelder: Bayer Technology Services GmbH, 51368 Leverkusen (DE)
(72) Erfinder: Tellmann, Kilian, 60385, Frankfurt am Main (DE); Mleczko, Leslaw, 41542, Dormagen (DE); Laue, Stephan, 2018, Antwerpen (BE); Haverkamp, Verena, 51467, Bergisch Gladbach (DE); Grünewald, Marcus, 44309, Dortmund (DE)

(57) **Zusammenfassung**

Die Erfindung betrifft ein Verfahren zur kontinuierlichen Oxidation von Thioethern. Das Verfahren eignet sich insbesondere zur selektiven Herstellung von Sulfoxiden oder Sulfonen.

## Beschreibung

Die Erfindung betrifft ein Verfahren zur kontinuierlichen Oxidation von Thioethern. Das Verfahren eignet sich insbesondere zur selektiven Herstellung von Sulfoxiden oder Sulfonen.

EP0623640A1 beschreibt eine zweistufige Oxidation von Polyarylensulfiden, wobei in erster Stufe ein Polyarylensulfid in einem Suspensionsmedium mit Ozon oxidiert wird und das erhaltene Reaktionsprodukt in einer zweiten Stufe in Essigsäure mit Wasserstoffperoxid in Gegenwart von katalytischen Mengen an konzentrierter Schwefelsäure und/oder unter Zusatz von Gleichgewichtspersäuren umgesetzt wird. Durch das zweistufige Verfahren werden die Thioethergruppen der Ausgangsverbindungen teilweise oder vollständig in Sulfoxid und/oder Sulfongruppen überführt. Der Anteil der Sulfongruppen in der resultierenden Verbindung ist abhängig von der Menge des Oxidationsmittels, das in der zweiten Stufe angewandt wird. Durch das in dieser Patentanmeldung beschriebene Verfahren wird eine Raum-Zeit-Ausbeute (R.Z.A.) von maximal 35 kg/(h*m³) (vergleiche die Werte von Beispiel 3 der EP0623640A1; Formel zur Berechnung der R.Z.A. auf Seite 8: 0,0061 kg Produktausbeute / (≥ 3,5 h Reaktorbetriebszeit * ≥ 0,000050 m³ Reaktorfüllvolumen) erreicht.

EP0623641A1 beschreibt ein Verfahren zur Herstellung eines Polyarylensulfons durch Oxidation eines Polyarylenthioethers, wobei die Umsetzung in Essigsäure mit Wasserstoffperoxid in Gegenwart von katalytischen Mengen an konzentrierter Schwefelsäure und/oder in Essigsäure unter Zusatz von Gleichgewichtspersäuren erfolgt. Die Reaktionstemperatur liegt im Bereich von 40 bis 90°C und die Reaktionszeit beträgt 0,5 bis 12 Stunden. Durch das in dieser Patentanmeldung beschriebene Verfahren wird eine Raum-Zeit Ausbeute von maximal 10 kg/(h*m³) (vergleiche die Werte von Beispiel 1 der EP0623641A1; Formel zur Berechnung der R.Z.A. auf Seite 8: 0,0071 kg Produktausbeute / (≥ 16 h Reaktorbetriebszeit * ≥ ,000048 m³ Reaktorfüllvolumen) erreicht.

EP0903174A1 offenbart unter anderem ein Verfahren zur kontinuierlichen Oxidation von organischen Sulfiden zu Sulfonen unter Verwendung von Wasserstoffperoxid als Oxidationsmittel und dem Einsatz eines Mikroreaktors, welcher über eine Schar von parallelen Reaktionskanälen und benachbarten Kühlkanälen verfügt. Durch das in dieser Patentanmeldung beschriebene Verfahren wird eine Raum-Zeit Ausbeute von ca. 72.000 kg/(h*m³) kg/(h*m³) (vergleiche die Werte von Beispiele 3 der EP0903174A1; Formel zur Berechnung der R.Z.A. auf Seite 8: 37,62 kg/h Gesamtmassenstrom am Reaktoreingang * 2,7/(2,7 + 1) Anteil Eduktstrom 6a am Gesamtmassenstrom * 5,33 kg/(5,33 kg + 8,76 kg) Anteil Edukt am Eduktstrom 6a * (0,316 kg/mol / 0,252 kg/mol) Verhältnis der Molmassen * 88,2% Produktausbeute / (ca. 0,00016 m³ Reaktorvolumen) erreicht. Nachteilig bei diesem Verfahren ist jedoch die Produktausbeute, die bei maximal 88,2% liegt (vgl. Bsp. 3).

Aus dem Stand der Technik sind demnach Verfahren zur kontinuierlichen Oxidation von Thioethern zur Herstellung von Sulfoxiden oder Sulfonen bekannt. Hohe Produktausbeuten sind mit diesen Verfahren möglich, werden jedoch stets nur mit niedrigeren Raum-Zeit-Ausbeuten erreicht. Verfahren die eine hohe Raum-Zeit-Ausbeute ermöglichen, bringen jedoch wiederum Nachteile bezüglich der Produktausbeute mit sich. Darüber hinaus ergeben sich bei adiabat oder quasi-adiabat geführten Verfahren mit hohen Raum-Zeit-Ausbeuten erhebliche Sicherheitsproblematiken. Speziell beim Einsatz von Oxidationsmitteln wie z.B. Wasserstoffperoxid oder Persäuren findet mit zunehmend hoher Reaktionstemperatur eine Zersetzung dieser Verbindungen unter Freisetzung von Sauerstoff statt, was in Kombination mit Dämpfen von brennbaren Stoffen zu explosiven Gemischen führen kann. Die Zersetzung des Oxidationsmittels verläuft darüber hinaus exotherm und steigert die Reaktorinnentemperatur also zusätzlich in Richtung gefährlicher Bereiche. Ein weiterer Nachteil bekannter Verfahren sind die geringen Produktselektivitäten speziell im Verhältnis von Sulfoxid zu Sulfon.

Die Aufgabe der vorliegenden Erfindungen liegt deshalb in der Bereitstellung eines Verfahrens zur kontinuierlichen Oxidation von Thioethern, insbesondere zur selektiven Herstellung von Sulfoxiden oder Sulfonen, welche die Nachteile der im Stand der Technik beschriebenen Verfahren adressiert und überwindet.

Gelöst wird die Aufgabe in einer ersten Ausführungsform der Erfindung überraschenderweise durch ein Verfahren zur kontinuierlichen Oxidation von Thioethern, insbesondere zur selektiven Herstellung von Sulfoxiden oder Sulfonen, welches folgende Schritte aufweist:
a) Mischen eines Thioethers und eines Oxidationsmittels in einer ersten Reaktionszone,
b) Überführung des Reaktionsgemisches aus Schritt a) in eine zweite Reaktionszone, wobei die zweite Reaktionszone isotherm betrieben wird.

Der eingesetzte Thioether ist insbesondere ein aromatischer Thioether vorzugsweise mit der Formel Ar(SR)ₙ, worin n = 1 oder mehr bedeutet; R unsubstituiert oder substituiert sein kann und ausgewählt ist aus der Gruppe von C₁- bis C₂₀-Alkyl, C₃- bis C₁₂-Cycloakl, C₃- bis C₂₀-Alkenyl, C₃- bis C₂₀-Alkynyl, Aryl und Ar für einen substituierten oder unsubstituierten aromatischen Rest vorzugsweise ausgewählt aus der Gruppe von Benzol, Naphthalin und Biphenyl steht. Erfindungsgemäß bevorzugt wird ein unsubstituiertes oder substituiertes Thioanisol als Edukt eingesetzt. Substituenten sind insbesondere ausgewählt aus der Gruppe von C₁- bis C₂₀-Alkyl, Halogen, C₁- bis C₂₀-Methoxy und -CN.

Der Thioether wird vorzugsweise vor der Mischung mit dem Oxidationsmittel in einer Carbonsäure wie beispielsweise Essigsäure, Ameisensäure, Benzoesäure, *meta*-Chlorbenzoesäure, Adipinsäure, Ölsäure, Buttersäure, Zitronensäure, Acrylsäure und vorzugsweise Essigsäure gelöst. Um die Gleichgewichtseinstellung zu beschleunigen, kann in katalytischen Mengen Schwefelsäure dazugegeben werden. Neben der Schwefelsäure können auch andere geeignete Säuren verwendet werden. Alternativ können Persäuren auch direkt als Oxidationsmittel verwendet werden, einfacher ist jedoch häufig deren *in situ* Herstellung.

Vorzugsweise wird für das erfindungsgemäße Verfahren zum einen eine angesäuerte Lösung von 5 bis 50 Gew.-%, vorzugsweise 20 Gew.-% Thioether und 0 bis 20 Gew.-%, vorzugsweise 1 bis 10 Gew.-% Schwefelsäure in einer aliphatischen Monocarbonsäure, zum anderen eine 10 bis 50 Gew.-%, vorzugsweise 35 Gew.-% Wasserstoffperoxid-Lösung verwendet, die dann am Mischpunkt miteinander vermischt werden. Am Mischpunkt sollte vorzugsweise ein Oxidationsmittel zu Thioether (mol/mol)-Mindestverhältnis von etwa 1/1 vorliegen, um mit dem erfindungsgemäßen Verfahren den Ausgangsstoff Thioether weitestgehend umzusetzen und Sulfoxide selektiv herzustellen zu können. Für die selektive Herstellung von Sulfonen sollte beim erfindungsgemäßen Verfahren vorzugsweise ein Oxidationsmittel zu Thioether (mol/mol)-Mindestverhältnis von etwa 2/1 vorliegen.

Bei der selektiven Herstellung von Sulfoxiden hat es sich überraschenderweise zusätzlich als vorteilhaft erwiesen, wenn der Lösung zusätzlich noch ein Phasentransferkatalysator dazugegeben wird. Erfindungsgemäß bevorzugt wird als Phasentransferkatalysator eine quartäre Ammoniumverbindung verwendet. Besonders geeignet sind Trialkylmethylammoniumsalze wie z.B. Aliquat© 336 (Cognis). Die oben beschriebene Lösung mit dem Thioether, der Persäure und der aliphatischen Monocarbonsäure enthält in dieser Ausführungsform der Erfindung dann zusätzlich vorzugsweise etwa 1 Gew.-% eines Phasentransferkatalysators. Am Mischpunkt bzw. in dem aus der Vereinigung der Eduktströme resultierenden Gesamtgemisch sollte vorzugsweise eine Konzentration des Phasentransferkatalysators von etwa 1,0 Gew.-% vorliegen.

Das Oxidationsmittel wird ausgewählt aus der Gruppe von Sauerstoffdifluorid; Xenondifluorid; Kryptondifluorid; Wasserstoffperoxid H₂O₂ und dessen Addukte z.B. mit Harnstoff sowie dessen Derivate wie Natriumpercarbonat, Natriumpersulfat; sauerstoffhaltige Anionen (Oxoanionen) von Übergangsmetallen in hohen Oxidationsstufen wie Permanganat MnO₄⁻ oder Dichromat Cr₂O₇²⁻ und Chrom(VI)-oxid (Jones-Oxidation); Metallionen wie Ce⁴⁺, Edelmetallionen wie die von Kupfer, Silber, Palladium, Platin, Rhodium und Gold, Anionen von Halogensauerstoffsäuren wie Bromat BrO₃⁻ , Hypochlorit C10⁻ , Chlorat ClO₃⁻ und Perchlorat ClO₄⁻; Sauerstoff, Schwefel, Fluor, Chlor, Brom und Iod sowie alle atomaren/molekularen Modifikationen bzw. Allotrope der vorgenannten Stoffe. Das Oxidationsmittel liegt vorzugsweise in flüssiger Form, z.B. als Lösung, vor. Erfindungsgemäß bevorzugt ist die Verwendung von Wasserstoffperoxid (H₂O₂) als Oxidationsmittel. Wasserstoffperoxid wird insbesondere in Form einer 10 ― 50%igen wässrigen Lösung, vorzugsweise einer 35%igen wässrigen Lösung eingesetzt.

Das Mischen des Thioethers bzw. der oben beschriebenen Lösung mit dem Oxidationsmittel bzw. dessen oben beschriebenen Lösung in einer ersten Reaktionszone findet kontinuierlich statt. Als vorteilhaft hat sich die Verwendung eines Mikromischers und vorzugsweise eines statischen Mikromischers erwiesen. Für das erfindungsgemäße Verfahren können alle bekannten statischen Mikromischer eingesetzt werden. In einer bevorzugten Ausführungsform der Erfindung werden dabei als statische Mischer Multilaminationsmischer, "Teilen und Zusammenfügen"-Mischer ("split and recombine"-Mischer) oder auch Mischer mit einer Querschnittsverengung eingesetzt. Bevorzugt verwendet werden statische Mischer, die kontinuierlich durchströmt werden. Darunter fallen beispielsweise Stapelmischer (gemäß DE20206371U1) oder Schlitzplattenmischer (gemäß WO 2004052518A2) oder auch Kammmischer (gemäß DE20209009 U1), wie sie z. B. bei Ehrfeld Mikrotechnik BTS GmbH angeboten werden. Hierbei handelt es sich um Multilaminationsmischer, bei denen die beiden zu vermischenden Fluidströme in eine Vielzahl von dünnen Lamellen oder Filmen aufgefächert werden und diese Lamellen anschließend abwechselnd ineinander gekämmt werden, so dass es durch Diffusion und Sekundärströmungen zu einer schnellen Vermischung kommt. Ebenfalls möglich sind V-Typ-Mischer (z.B. vom Forschungszentrum Karlsruhe), "Teilen und Zusammenfügen"-Mischer ("split and recombine"-Mischer) wie z. B. Kaskadenmischer oder Rautenmischer (z.B. von Ehrfeld Mikrotechnik BTS GmbH) oder Caterpillarmischer (z.B. vom Institut für Mikrotechnik Mainz) in dem die zu vermischenden Produktströme in kleinere Ströme aufgeteilt und diese kleinen Ströme wiederholt zusammengeführt und aufgeteilt werden, oder auch Mischer mit einer Querschnittsverengung, wie Fokusmischer oder Zyklonmischer, oder auch Jetmischer (gemäß EP1165224B1), z. B. erhältlich bei der Firma Synthesechemie und Prallstrahlmischer oder Ventilmischer (wie in WO 2005/079964 A1 beschrieben) erhältlich bei Ehrfeld Mikrotechnik BTS GmbH. In einer bevorzugten Ausführungsform der Erfindung werden Mikromischer mit charakteristischen Dimensionen von kleiner als 1000 µm, bevorzugt kleiner 500 µm, besonders bevorzugt kleiner 200 µm eingesetzt. In derartige Mikromischer werden die Edukte (d.h. das Oxidationsmittel und der Thioether) vorzugsweise mittels präziser und pulsationsarmer Pumpen, wie z. B. HPLC-Pumpen oder Spritzenpumpen, zudosiert. Besonders bevorzugt ist die Verwendung der statischen Mikromischer LH2, LH25 und/oder einem Kaskadenmischer (alle von Ehrfeld Mikrotechnik BTS GmbH).

In einem weiteren Gegenstand der vorliegenden Erfindung wird das erfindungsgemäße Verfahren in einem statischen Mikromischer durchgeführt und das Massenstromverhältnis zwischen Thioether (bzw. der oben beschriebenen Lösung) und Oxidationsmittel (bzw. einer Oxidationsmittellösung) im Bereich von etwa 3:1 bis 6:1 eingestellt. Bei der Herstellung von Sulfoxiden beträgt das Massenstromverhältnis zwischen Thioether (bzw. der oben beschriebenen Lösung) und Oxidationsmittel (bzw. einer Oxidationsmittellösung) insbesondere mit den Mikromischern LH2, LH25 oder Kaskadenmischer (alle von Ehrfeld Mikrotechnik BTS GmbH) vorzugsweise etwa 6:1. Bei der selektiven Herstellung von Sulfon beträgt das Massenstromverhältnis zwischen Thioether (bzw. der oben beschriebenen Lösung) und Oxidationsmittel (bzw. einer Oxidationsmittellösung) insbesondere mit den Mikromischern LH2, LH25 oder Kaskadenmischer (alle von Ehrfeld Mikrotechnik BTS GmbH) vorzugsweise etwa 3:1. Die Verhältnisse der Massenströme bestimmen, zusammen mit der Konzentration der Edukte/Reaktanden in den jeweiligen Einsatzlösungen, die Stöchiometrie von Oxidationsmittel zu Thioether; daher ist eine exakte Einhaltung der bevorzugten Werte vorteilhaft. Dies lässt sich technisch z.B. über Massendurchflussmesser gekoppelt mit einer entsprechend geeigneten Prozesssteuerungstechnik erreichen.

Auf Grund der Verwendung eines Mikromischers wird das Oxidationsmittel mit dem Thioether bzw. der oben beschriebenen Lösungen dieser Stoffe effizient und schnell gemischt. Die Mischzeit liegt vorzugsweise bei weniger als 1 Sekunde, da in diesem Zeitraum bereits ein nennenswerter Umsatz des jeweiligen Edukts und gegebenenfalls sogar schon des Zwischenprodukts stattfinden kann und Inhomogenitäten im Reaktionsgemisch in vielen Fällen zu Selektivitätseinbußen führen können. Die Mischzeit beschreibt erfindungsgemäß diejenige Zeit, in welcher der Thioether und das Oxidationsmittel in der ersten Reaktionszone verbleiben.

Der Mischprozess wird vorzugsweise beim vorliegenden Verfahren in einem Temperaturbereich von 0 °C bis 120 °C, vorzugsweise 30°C bis 90°C durchgeführt. Bei der selektiven Herstellung von Sulfoxiden wird das Mischen insbesondere bei einer Temperatur von 40°C bis 60 °C und vorzugsweise bei einer Temperatur von 45°C bis 55 °C und noch bevorzugter bei etwa 50 °C durchgeführt. Bei der selektiven Herstellung von Sulfonen wird das Mischen insbesondere bei einer Temperatur von 60°C bis 80°C und vorzugsweise bei einer Temperatur von 65°C bis 75 °C und noch bevorzugter bei etwa 70 °C durchgeführt. Aufgrund der kurzen Mischzeit bleibt die Temperatur in der ersten Reaktionszone im Wesentlichen konstant. Im Wesentlichen konstant heißt, dass die Temperaturunterschiede innerhalb der ersten Reaktionszone nicht größer als 5°C und besonders bevorzugt nicht größer als 1 °C sind.

Nach dem Mischen wird das Reaktionsgemisch in eine zweite Reaktionszone überführt, wobei diese zweite Reaktionszone isotherm betrieben wird. Vorzugsweise befindet sich die zweite Reaktionszone direkt hinter der ersten Reaktionszone (d.h. vorzugsweise hinter dem Mikromischer) und umfasst vorzugsweise eine Anordnung von mikrostrukturierten Wärmetauschern und ggfs. Verweilerstrecken mit einer bestimmten Verweilzeit. Bevorzugt ist die Anordnung so gestaltet, dass der Temperaturverlauf im strömenden Reaktionsgemisch entlang der Strömungsrichtung durch die Abfolge von Wärmetauschern und ggfs. vorhandenen Verweilerstrecken isotherm einstellbar ist. Für das erfindungsgemäße Verfahren werden vorzugsweise alle bekannten mikrostrukturierten Wärmetauscher eingesetzt. Bekannt sind Mikro-Wärmetauscher, die aus gestapelten, mit vielen Mikrokanälen versehenen und miteinander verschweißten oder anders miteinander verbundenen dünnen Platten bestehen. Die Geometrie solcher Wärmetauscher kann illustrativ beispielsweise als annähernd würfelförmig beschrieben werden, wobei bei einer Kantenlänge von beispielsweise 3 cm Wärmeübertragungsleistungen im Bereich von 100 kW erzielt werden können, wie dies beispielhaft beschrieben ist in (2nd, International Conference an Microreaction Technology, Tropical Conference Preprints, (ISBN 0-8169-9945-7, S. 88―95). Solche mikrostrukturierten Wärmetauscher sind z. B. Kreuzstrom- oder Gegenstrom-Plattenwärmetauscher, wie in DE 37 09 278 A1 beschrieben, und wie sie z. B. bei der Ehrfeld Mikrotechnik BTS GmbH erhältlich sind. Muss mit dem Auftreten von Partikeln gerechnet werden, so kommen bevorzugt mikrostrukturierte Wärmetauscher zum Einsatz, deren engste Durchlassstelle so breit ist, dass die Partikel ungehindert passieren können. Ein solcher Wärmetauscher ist beispielsweise das Rohr-Temperiermodul der Ehrfeld Mikrotechnik BTS GmbH. Ein bevorzugt eingesetzter mikrostrukturierter Wärmetauscher für das erfindungsgemäße Verfahren ist der Sandwichreaktor der Firma Ehrfeld Mikrotechnik BTS GmbH. Eine isotherme Fahrweise kann im Voraus durch Simulation der Wärmeströme ― auf Basis einer vereinfachten Reaktionskinetik und bekannter thermodynamischer Grunddaten ― und der Verwendung dieser für die Reaktorauslegung bzw. das Apparatedesign, berechnet werden. Überprüft werden die Annahmen dann in der Praxis durch mehrere Temperaturmessstellen entlang des Reaktorverlaufs.

In einer bevorzugten Ausführungsform des erfindungsgemäßen Verfahrens werden die Einsatzlösungen kontinuierlich in einem Mikromischer durchmischt und die Reaktionsmischung wird dann unmittelbar einer zweiten Reaktionszone zugeführt, in der sie mittels Wärmetauscher und gegebenenfalls vorhandenen Verweilerstrecken isotherm gefördert werden kann.

Die Reaktion in der zweiten Reaktionszone wird vorzugsweise bei einer bestimmten Temperatur im Temperaturbereich von 20°C bis 100°C durchgeführt. Bei der selektiven Herstellung von Sulfoxiden wird die Temperatur in der zweiten Reaktionszone isotherm bei einer bestimmten Temperatur vorzugsweise im Temperaturbereich von 40°C bis 60°C und vorzugsweise bei etwa 50 °C gehalten. Bei der selektiven Herstellung von Sulfonen wird die Temperatur in der zweiten Reaktionszone isotherm bei einer bestimmten Temperatur vorzugsweise im Temperaturbereich von 60°C bis 80°C und vorzugsweise bei etwa 70°C gehalten.

Die hydrodynamische Verweilzeit des Reaktionsgemisches in der zweiten Reaktionszone liegt bei der Verwendung von den oben beschriebenen Wärmetauschern und Reaktionstemperaturen vorzugsweise bei 2 bis 15 Minuten, noch bevorzugter bei 3 bis 8 Minuten und besonders bevorzugt bei etwa 5 Minuten. Die hydrodynamische Verweilzeit entspricht näherungsweise der reinen Reaktionszeit und über letztere wird der Umsatz pro Zeit und damit die Produktbildungsrate maßgeblich beeinflusst. Durch eine geeignete Einstellung der hydrodynamischen Verweilzeit kann gelegentlich auch die Bildung von Folgeprodukten erfolgreich vermieden werden. Die hydrodynamische Verweilzeit (= tau_hydro) ergibt sich aus der Beziehung tau_hydro = Reaktorvolumen [m³] / Volumenstrom [m³/h] = Reaktorvolumen [m³] / Massenstrom [kg/h] * mittlere Dichte [kg/m³].

Isotherm betrieben im Sinne der vorliegenden Erfindung bedeutet, dass die Temperaturunterschiede innerhalb der zweiten Reaktionszone nicht größer als 10°C und besonders bevorzugt nicht größer als 5°C sind.

In einer weiteren Ausführungsform sind alle Reaktionszonen in einer gemeinsamen Reaktorhülle aufgenommen.

Vorteil des erfindungsgemäßen Verfahrens ist, dass der eingesetzte Thioether bei der kontinuierlichen Oxidation sowohl im Wesentlichen vollständig und mit sehr hoher Selektivität umgesetzt wird, als auch dabei aufgrund der hohen R.Z.A. zugleich ein vergleichsweise kleiner und kompakter Reaktor zum Einsatz kommen kann und das gesamte Verfahren dabei außerdem noch mit hoher Sicherheit zu betreiben ist; überraschend ist auch, dass die Bedingungen aus dem Labormaßstab direkt in den Pilotmaßstab übertragbar sind und dass der meist mit einer Maßstabsvergrößerung einhergehende, zusätzliche Entwicklungsaufwand und die häufig damit verbundenen Zeitverluste entfallen. Im Wesentlichen vollständig umgesetzt bedeutet dabei, dass der Umsatz größer als 98%, bevorzugt größer 99 % ist. Im Wesentlichen mit sehr hoher Selektivität umgesetzt bedeutet dabei, dass wegen der Kombination aus hohen Umsätzen mit sehr hohen Selektivitäten sehr hohe Produktausbeuten erzielt werden, die im Falle der kontinuierlichen Oxidation zum Sulfoxid bzw. Sulfon bei mindestens 98%, bevorzugt bei größer als 98% liegen. Eine hohe R.Z.A. bedeutet dabei einen Wert von größer 100 kg_{produkt}/(h*m³), bevorzugt von größer 1.000 kg_{produkt}/(h*m³). Eine hohe Sicherheit bedeutet dabei, dass am Reaktorausgang (d.h. nach der zweiten Reaktionszone, vorzugsweise dem mikrostrukturierten Wärmetauscher) keine bei Raumtemperatur kondensierbare Gasphase festgestellt wird, besonders bevorzugt dass weder im Reaktor (d.h. in der zweiten Reaktionszone, vorzugsweise dem mikrostrukturierten Wärmetauscher) noch am Reaktorausgang eine bei Raumtemperatur kondensierbare Gasphase festgestellt werden kann. Unter einer Maßstabsvergrößerung (z.B. von Labormaßstab zur Pilotanlage) wird eine Steigerung des Durchsatzes [kg_{produkt}/h] um größer als das 5-fache, bevorzugt größer als das 50-fache, erachtet.

Der technischen Ausdruck einer Reaktorproduktivität wird mit der Raum-Zeit-Ausbeute R.Z.A. {engl. space-time-yield, S.T.Y.} beschrieben und nach folgender Formel ermittelt:
R.Z.A. = Produktausbeute [kg] / (Zeit des Reaktorbetriebs [h] * benötigtes Reaktorvolumen [ₘ3]) =
= Edukteinsatz [kg] * Produktausbeute [mol/mol] * (Molmasse Produkt [kg/mol] molmasse Edukt [kg/mol]) / (Zeit des Reaktorbetriebs [h] * benötigtes Reaktorvolumen [m³]) =
= Edukteinsatz [kg] * Eduktumsatz [mol/mol] * Produktselektivität [mol/mol] * (Molmasse Produkt [kg/mol] / Molmasse Edukt [kg/mol]) / (Zeit des Reaktorbetriebs [h] * benötigtes Reaktorvolumen [m³]).

### Beispiele

### Beispiel 1: Kontinuierliche Sulfoxid-Herstellung am Beispiel des Methylphenylsulfoxids im Labormaßstab, Variante 1

Es wurde eine angesäuerte Lösung von Thioanisol in Essigsäure mit folgender Zusammensetzung hergestellt: 20 Gew.-% Thioanisol, 1,16 Gew.-% konzentrierte Schwefelsäure, Rest Essigsäure (Lösung A1). Als Oxidationsmittel wurde 35 Gew.-% Wasserstoffperoxid in Wasser eingesetzt (Lösung B). Diese beiden Lösungen wurden kontinuierlich in einem mikrostrukturierten statischen Mischer (Ehrfeld Mikrotechnik BTS GmbH; Modelle LH2 und Kaskadenmischer) bei 50°C vermischt, das Massenstromverhältnis von Lösung A1 zu Lösung B betrug 6,09/1 kg/kg (1,05 mol/mol Wasserstoffperoxid/Thioanisol). Die Mischzeit lag dabei bei <1 s. Direkt hinter dem Mikromischer strömt das Reaktionsgemisch dann in eine Reihe von Wärmetauschern, die ausreichend Reaktionswärme abführen können, um eine isotherme Fahrweise zu gewährleisten. Die hydrodynamische Verweilzeit des Gemischs in den Wärmetauschern lag bei insgesamt 5,0 min. Am Reaktorausgang kann das Reaktionsgemisch mittels Auffangen in einer gerührten, verdünnten wässrigen Natriumthiosulfat-Lösung zügig auf Raumtemperatur abgekühlt werden, dabei wird auch der kleine verbleibende Restüberschuss an Wasserstoffperoxid abgequencht.

Analysen von Proben aus dem Produktstrom mittels HPLC zeigen, dass am Reaktorausgang Thioanisol kontinuierlich zu 99% umgesetzt wird, mit Selektivitäten S1 (Thioanisol=>Sulfoxid) >99% und S2 (Thioanisol=>Sulfon) <1%. Demnach lässt sich nach diesem Verfahren Thioanisol kontinuierlich zu Methylphenylsulfoxid mit Ausbeuten von >98% umsetzen. Die Oxidationsselektivität (Sulfoxid/Sulfon) beträgt bei diesem Verfahren >99/1, der bei einem nahezu vollständigen Thioanisolumsatz erzielt wird.

Diese hochselektive Umsetzung führt, in Kombination mit der kurzen hydrodynamischen Verweilzeit, zu einer hohen spezifischen Produktivität, ausgedrückt in einer Raum-Zeit-Ausbeute von etwa 1900 kg/(h*m³). Auf diesem Wege wurden im Labormaßstab 27 g/h Methylphenylsulfoxid hergestellt.

Demnach werden kleine, kompakte Reaktoren für die gewünschte Umsetzung genügen. Darüber hinaus kann die stark exotherme Umsetzung (ΔH_{R} ca. ―250 kJ/mol) aufgrund der isothermen Fahrweise trotz verhältnismäßig hoher Reaktionstemperatur (50°C) sicher durchgeführt werden, da eine thermische Zersetzung des Wasserstoffperoxids bei diesen Temperaturen und der kurzen Reaktionszeit nicht auftritt. Eine Zersetzung des Wasserstoffperoxids setzt unter anderem Sauerstoff frei, der in Kombination mit vielen organischen Einsatzstoffen (auch den hier verwendeten) explosive Gemische erzeugen würde. Zudem ist die Zersetzung des Wasserstoffperoxids ebenfalls eine exotherme Reaktion (ΔH_{z} ca. ―110 kJ/mol), deren Reaktionswärme mittels zusätzlicher Erwärmung des gesamten Reaktionsgemisches die thermische Wasserstoffperoxidzersetzung weiter beschleunigen könnte. Dieses Gefahrenpotenzial wird dank isothermer Fahrweise jedoch vollständig umgangen.

### Beispiel 2: Kontinuierliche Sulfoxid-Herstellung am Beispiel des Methylphenylsulfoxids im Labormaßstab, Variante 2

Die Vorgehensweise entsprach jener des Beispiels 1, mit Ausnahme der Lösung A1, die durch folgende Lösung ersetzt wurde: 20 Gew.-% Thioanisol, 1,16 Gew.-% konzentrierte Schwefelsäure, 1,16 Gew.-% Aliquat 336 (Methyltri-*n*-octylammoniumchlorid), Rest Essigsäure (Lösung A2). Bei dieser Vorgehensweise werden etwas stabilere Messergebnisse als nach Variante 1 erhalten. Analysen von Proben aus dem Produktstrom mittels HPLC zeigen, dass am Reaktorausgang Thioanisol kontinuierlich zu 99% umgesetzt wird, mit Selektivitäten S1 (Thioanisol=>Sulfoxid) >99% (typischerweise 99,0-99,5%) und S2 (Thioanisol=>Sulfon) <1% (typischerweise 1,0-0,5%). Auf diesem Wege wurden im Labormaßstab 27 g/h Methylphenylsulfoxid hergestellt.

### Beispiel 3: Kontinuierliche Sulfoxid-Herstellung am Beispiel des Methylphenylsulfoxids im Pilotmaßstab

Die Lösungen A2 und B wurden wie im Beispiel 2 hergestellt und miteinander kontinuierlich zur Reaktion gebracht. Auf diesem Wege wurden im Pilotmaßstab 2,9 kg/h Methylphenylsulfoxid hergestellt. Analysen von Proben aus dem Produktstrom mittels HPLC zeigen, dass am Reaktorausgang Thioanisol kontinuierlich zu >99,5% umgesetzt wird, mit Selektivitäten S1 (Thioanisol=>Sulfoxid) von 98,9% und S2 (Thioanisol=>Sulfon) von 1,1%. Demnach lässt sich nach diesem Verfahren Thioanisol kontinuierlich zu Methylphenylsulfoxid mit Ausbeuten von >98,5% umsetzen. Diese hochselektive Umsetzung führt, in Kombination mit hydrodynamischen Verweilzeiten wie im Labormaßstab, zu einer hohen spezifischen Produktivität, ausgedrückt in einer Raum-Zeit-Ausbeute von etwa 1900 kg/(h*m³).

### Beispiel 4: (Vergleichsbeispiel) Diskontinuierliche Sulfoxid-Herstellung am Beispiel des Methylphenylsulfoxids im Labormaßstab als Vergleichsbeispiel: Dosierkontrollierte (semibatch) Fahrweise

In einem drei- oder vierhalsigen Rundkolben (500 mL) wurden 148 mL der Lösung A1 vorgelegt, mit Stickstoff überschleiert und unter Rühren auf Reaktionstemperatur gebracht. Diese wurde auf (a) 30°C bzw. (b) 50°C eingestellt. Unter Rühren wurden dann die Lösung B (Wasserstoffperoxid 35% in Wasser) so zügig wie möglich zugegeben, aber mit der Vorgabe, dass die Reaktionstemperatur dabei um nicht mehr als 5°C überschritten wird (quasiisotherme Fahrweise). Auf diese Weise wurden 1,00 Äquivalente Wasserstoffperoxid zudosiert und eine Probe gezogen. Im Anschluss wurden auf die gleiche Weise weitere 0,05 Äquivalente Wasserstoffperoxid zudosiert (insgesamt nun 1,05 Äquivalente) und eine weitere Probe gezogen.

Dabei wurden folgenden Umsätze und Selektivitäten S1 (Methylphenylsulfoxid, erwünscht) und S2 (Methylphenylsulfon, unerwünscht) erzielt:
(a) Innentemperatur 30°C bis maximal 35°C
   1,00 Äquivalente Wasserstoffperoxid:
      Umsatz Thioanisol >99%, Selektivität S1 = 94,8%, Selektivität S2 = 5,2%;
   1,05 Äquivalente Wasserstoffperoxid:
      Umsatz Thioanisol >99%, Selektivität S1 = 89,1%, Selektivität S2 = 10,9%;
(b) Innentemperatur 50°C bis maximal 55°C
   1,00 Äquivalente Wasserstoffperoxid:
      Umsatz Thioanisol = 99,1%, Selektivität S1 = 94,9%, Selektivität S2 = 5,1%;
   1,05 Äquivalente Wasserstoffperoxid:
      Umsatz Thioanisol = 97,9%, Selektivität S1 = 88,3%, Selektivität S2 = 11,7%;

### Beispiel 5: Kontinuierliche Sulfoxid-Herstellung am Beispiel des Methyl(2-fluor)phenyl-sulfoxids im Labormaßstab

Die Vorgehensweise entsprach jener des Beispiels 2, mit Ausnahme der Tatsache, dass Lösung A 20 Gew.-% 2-Fluorthioanisol anstelle 20 Gew.-% Thioanisol enthielt. Analysen von Proben aus dem Produktstrom mittels HPLC zeigen, dass am Reaktorausgang 2-Fluorthioanisol kontinuierlich mit Selektivitäten S1 (Thioether=>Sulfoxid) >99% und S2 (Thioether =>Sulfon) <1% umgesetzt wird.

### Beispiel 6: Kontinuierliche Sulfoxid-Herstellung am Beispiel des Methyl(3-methoxy)phenylsulfoxids im Labormaßstab

Die Vorgehensweise entsprach jener des Beispiels 2, mit Ausnahme der Tatsache, dass Lösung A 20 Gew.-% 3-Methoxythioanisol anstelle 20 Gew.-% Thioanisol enthielt. Analysen von Proben aus dem Produktstrom mittels HPLC zeigen, dass am Reaktorausgang 3-Methoxythioanisol kontinuierlich mit Selektivitäten S1 (Thioether=>Sulfoxid) >99% und S2 (Thioether =>Sulfon) <1% umgesetzt wird.

### Beispiel 7: Kontinuierliche Sulfoxid-Herstellung am Beispiel des Methyl(4-brom)phenylsulfoxids im Labormaßstab

Die Vorgehensweise entsprach jener des Beispiels 2, mit Ausnahme der Tatsache, dass Lösung A 20 Gew.-% 4-Bromthioanisol anstelle 20 Gew.-% Thioanisol enthielt. Analysen von Proben aus dem Produktstrom mittels HPLC zeigen, dass am Reaktorausgang 4-Bromthioanisol kontinuierlich mit Selektivitäten S1 (Thioether=>Sulfoxid) >99% und S2 (Thioether =>Sulfon) <1% umgesetzt wird.

### Beispiel 8: Kontinuierliche Sulfon-Herstellung am Beispiel des Methylphenylsulfons im Labormaßstab

Es wurde eine angesäuerte Lösung von Thioanisol in Essigsäure mit folgender Zusammensetzung hergestellt: 20 Gew.-% Thioanisol, 6,76 Gew.-% konzentrierte Schwefelsäure, Rest Essigsäure (Lösung A). Als Oxidationsmittel wurde 35 Gew.-% Wasserstoffperoxid in Wasser eingesetzt (Lösung B). Diese beiden Lösungen wurden kontinuierlich in einem mikrostrukturierten statischen Mischer (Ehrfeld Mikrotechnik BTS GmbH; LH2 und Kaskadenmischer) bei 70°C vermischt, das Massenstromverhältnis von Lösung A zu Lösung B betrug 3,04/1 kg/kg (2,10 mol/mol Wasserstoffperoxid/Thioanisol). Die Mischzeit lag dabei bei <1 s. Direkt hinter dem Mikromischer strömt das Reaktionsgemisch dann in eine Reihe von Wärmetauschern, die ausreichend Reaktionswärme abführen können, um eine isotherme Fahrweise zu gewährleisten. Die hydrodynamische Verweilzeit des Gemischs in den Wärmetauschern lag bei insgesamt 5,0 min. Am Reaktorausgang kann das Reaktionsgemisch mittels Auffangen in einer gerührten, verdünnten wässrigen Natriumthiosulfat-Lösung zügig auf Raumtemperatur abgekühlt werden, dabei wird auch der kleine verbleibende Restüberschuss an Wasserstoffperoxid abgequencht.

Analysen von Proben aus dem Produktstrom mittels HPLC zeigen, dass am Reaktorausgang Thioanisol kontinuierlich zu >99,9% umgesetzt wird, mit Selektivitäten S1 (Thioanisol=>Sulfoxid) <0,1% und S2 (Thioanisol=>Sulfon) >99,9%. Demnach lässt sich nach diesem Verfahren Thioanisol kontinuierlich zu Methylphenylsulfon mit Ausbeuten von >99,8% umsetzen. Die Oxidationsselektivität (Sulfoxid/Sulfon) beträgt bei diesem Verfahren >1000/1, der bei vollständigen Thioanisolumsatz erzielt wird.

Diese hochselektive Umsetzung führt, in Kombination mit der kurzen hydrodynamischen Verweilzeit, ausgedrückt in einer Raum-Zeit-Ausbeute von etwa 2400 kg/(h*m³). Auf diesem Wege wurden im Labormaßstab 74 g/h Methylphenylsulfon hergestellt.

Demnach werden kleine, kompakte Reaktoren für die gewünschte Umsetzung genügen. Darüber hinaus kann die stark exotherme Umsetzung (ΔH_{R} ca. ―500 kJ/mol) aufgrund der isothermen Fahrweise trotz verhältnismäßig hoher Reaktionstemperatur (70°C) sicher durchgeführt werden, da eine thermische Zersetzung des Wasserstoffperoxids bei diesen Temperaturen und der kurzen Reaktionszeit nicht auftritt. Eine Zersetzung des Wasserstoffperoxids setzt unter anderem Sauerstoff frei, der in Kombination mit vielen organischen Einsatzstoffen (auch den hier verwendeten) explosive Gemische erzeugen würde. Zudem ist die Zersetzung des Wasserstoffperoxids ebenfalls eine exotherme Reaktion (ΔH_{z} ca. ―110 kJ/mol), deren Reaktionswärme mittels zusätzlicher Erwärmung des gesamten Reaktionsgemisches die thermische Wasserstoffperoxidzersetzung weiter beschleunigen könnte. Dieses Gefahrenpotenzial wird dank isothermer Fahrweise jedoch vollständig umgangen.

### Beispiel 9: Kontinuierliche Sulfon-Herstellung am Beispiel des Methylphenylsulfons im Pilotmaßstab

Wie Beispiel 8, außer dass die Lösung A durch folgende Lösung ersetzt wurde: 20 Gew.-% Thioanisol, 1,16 Gew.-% konzentrierte Schwefelsäure, 1,16 Gew.-% Aliquat 336 (Methyltri-n-octylammoniumchlorid), Rest Essigsäure (Lösung A2). Auf diesem Wege wurden im Pilotmaßstab 2,6 kg/h Methylphenylsulfon hergestellt. Analysen von Proben aus dem Produktstrom mittels HPLC zeigen, dass am Reaktorausgang Thioanisol kontinuierlich zu >99,5% umgesetzt wird, mit Selektivitäten S1 (Thioanisol=>Sulfoxid) von 1,1% und S2 (Thioanisol=>Sulfon) von 98,9%. Demnach lässt sich nach diesem Verfahren Thioanisol kontinuierlich zu Methylphenylsulfon mit Ausbeuten von >98,5% umsetzen. Diese hochselektive Umsetzung führt, in Kombination mit hydrodynamischen Verweilzeiten wie im Labormaßstab, zu einer hohen spezifischen Produktivität, ausgedrückt in einer Raum-Zeit-Ausbeute von etwa 1900 kg/(h*m³).

### Beispiel 10 (Vergleichsbeispiel): Diskontinuierliche Sulfon-Herstellung am Beispiel des Methylphenylsulfons im Labormaßstab als Vergleichsbeispiel: Dosierkontrollierte (semibatch) Fahrweise

### In einem drei- oder vierhalsigen Rundkolben (500 mL) wurden 148 mL der Lösung A1 vorgelegt, mit Stickstoff überschleiert und unter Rühren auf Reaktionstemperatur gebracht. Diese wurde auf (a) 30°C bzw. (b) 50°C eingestellt. Unter Rühren wurden dann die Lösung B (Wasserstoffperoxid 35% in Wasser) so zügig wie möglich zugegeben, aber mit der Vorgabe, dass die Reaktionstemperatur dabei um nicht mehr als 5°C überschritten wird (quasiisotherme Fahrweise). Auf diese Weise wurden 2,10 Äquivalente Wasserstoffperoxid zudosiert, zwei Proben gezogen; eine 5 min nach Ende der Dosierung und eine weitere 30 min nach Ende der Dosierung.

Dabei wurden folgenden Umsätze und Selektivitäten S1(Methylphenylsulfoxid, unerwünscht) und S2(Methylphenylsulfon, erwünscht) erzielt:
(a) Innentemperatur 30°C bis maximal 35°C
   2,10 Äquivalente Wasserstoffperoxid: + 5 min
   Umsatz Thioanisol >99%, Selektivität S1 = 20,2%, Selektivität S2 = 79,8%;
   2,10 Äquivalente Wasserstoffperoxid: + 30 min
   Umsatz Thioanisol >99%, Selektivität S1 = 0,1%, Selektivität S2 = 99,9%;
(b) Innentemperatur 50°C bis maximal 55°C
   2,10 Äquivalente Wasserstoffperoxid: + 5 min
   Umsatz Thioanisol = 97,7%, Selektivität S1 = 6,3%, Selektivität S2 = 93,7%;
   2,10 Äquivalente Wasserstoffperoxid: + 30 min
   Umsatz Thioanisol >99%, Selektivität S1 < 0,1%, Selektivität S2 > 99,9%;

Die Selektivitäten sind teilweise sehr gut, die R.Z.A. dagegen aber schlecht. Ein Grund dafür ist die geringe Wärmeabfuhrleistung bei dieser diskontinuierlichen Sulfon-Herstellung.

### Beispiel 11: Kontinuierliche Sulfon-Herstellung am Beispiel des Methyl(2-fluor)phenylsulfons im Labormaßstab

Die Vorgehensweise entsprach jener des Beispiels 8, mit Ausnahme der Tatsache, dass Lösung A 20 Gew.-% 2-Fluorthioanisol anstelle 20 Gew.-% Thioanisol enthielt. Analysen von Proben aus dem Produktstrom mittels HPLC zeigen, dass am Reaktorausgang 2-Fluorthioanisol kontinuierlich zu >99,9% umgesetzt wird. Da das 2-Fluorthioanisol weniger reaktiv als Thioanisol ist, muss die Verweilzeit verlängert und/oder die Reaktionstemperatur erhöht werden, um eine Selektivität S2 von 99% zu erreichen.

### Beispiel 12: Kontinuierliche Sulfon-Herstellung am Beispiel des Methyl(3-methoxy)phenylsulfons im Labormaßstab

Die Vorgehensweise entsprach jener des Beispiels 8, mit Ausnahme der Tatsache, dass Lösung A 20 Gew.-% 3-Methoxythioanisol anstelle 20 Gew.-% Thioanisol enthielt. Analysen von Proben aus dem Produktstrom mittels HPLC zeigen, dass am Reaktorausgang 3-Methoxythioanisol kontinuierlich zu >99,9% umgesetzt wird. Da das 3-Methoxythioanisol weniger reaktiv ist als das Thioanisol, muss die Verweilzeit verlängert und/oder die Reaktionstemperatur erhöht werden, um eine Selektivität S2 von 99% zu erreichen.

### Beispiel 13: Kontinuierliche Sulfon-Herstellung am Beispiel des Methyl(4-brom)phenylsulfons im Labormaßstab

Die Vorgehensweise entsprach jener des Beispiels 8, mit Ausnahme der Tatsache, dass Lösung A 20 Gew.-% 4-Bromthioanisol anstelle 20 Gew.-% Thioanisol enthielt. Analysen von Proben aus dem Produktstrom mittels HPLC zeigen, dass am Reaktorausgang 4-Bromthioanisol kontinuierlich zu >99,9% umgesetzt wird. Eine Verweilzeitverlängerung und/oder eine Erhöhung der Reaktionstemperatur reicht aus, um eine Selektivität S2 von 99% zu erreichen.

## Patentansprüche

1. Verfahren zur kontinuierlichen Oxidation von Thioethern, welches folgende Schritte aufweist
a) Mischen eines Thioethers und eines Oxidationsmittels in einer ersten Reaktionszone,
b) Überführung des Reaktionsgemisches aus Schritt a) in eine zweite Reaktionszone, wobei die zweite Reaktionszone isotherm betrieben wird.

2. Verfahren nach Anspruch 1, **dadurch gekennzeichnet, dass** der Thioether ein aromatischer Thioether vorzugsweise ein unsubstituiertes oder substituiertes Thioanisol ist.

3. Verfahren nach einem der vorherigen Ansprüche, **dadurch gekennzeichnet, dass** die die Schritte a) und b) bei einer Temperatur von 0°C bis 120°C durchgeführt werden.

4. Verfahren nach einem der vorherigen Ansprüche, **dadurch gekennzeichnet, dass** der Thioether vor dem Mischen mit dem Oxidationsmittel in einer aliphatischen Monocarbonsäure und gegebenenfalls mit einer katalytischen Menge einer Schwefelsäure gelöst wird.

5. Verfahren nach Anspruch 4 zur Herstellung eines Sulfoxids, **dadurch gekennzeichnet, dass** der Lösung zusätzlich ein Phasentransferkatalysator hinzugefügt wird.

6. Verfahren nach einem der vorherigen Ansprüche, **dadurch gekennzeichnet, dass** die erste Reaktionszone ein Mikromischer ist.

7. Verfahren nach Anspruch 6, **dadurch gekennzeichnet, dass** die Mischzeit eines Thioethers und eines Oxidationsmittels im Mikromischer weniger als 1 Sekunde beträgt.

8. Verfahren nach Anspruch 6 und/oder 7, **dadurch gekennzeichnet, dass** im Mikromischer am Mischpunkt ein Oxidationsmittel zu Thioether (mol/mol)-Mindestverhältnis von etwa 1/1 vorliegt, wenn selektiv Sulfoxide hergestellt werden sollen, hingegen ein Mindestverhältnis von etwa 2/1 vorliegt, wenn selektiv Sulfone hergestellt werden sollen.

9. Verfahren nach einem der vorherigen Ansprüche, **dadurch gekennzeichnet, dass** die erste Reaktionszone einen mikrostrukturierten Mikromischer, vorzugsweise mit charakteristischen Dimensionen von kleiner als 1000 µm, umfasst.

10. Verfahren nach einem der vorherigen Ansprüche, **dadurch gekennzeichnet, dass** die hydrodynamische Verweilzeit des Reaktionsgemisches in der zweiten Reaktionszone bei 3 bis 8 Minuten, vorzugsweise bei etwa 5 Minuten liegt.
